Europäisches Patentamt

European Patent Office

Office européen des brevets

(11) Veröffentlichungsnummer: **0 067 834**
**B1**

(12) **EUROPÄISCHE PATENTSCHRIFT**

(19)

(45) Veröffentlichungstag der Patentschrift:
**02.05.85**

(21) Anmeldenummer: **82900096.7**

(22) Anmeldetag: **21.12.81**

(86) Internationale Anmeldenummer:
**PCT/DE 81/00231**

(87) Internationale Veröffentlichungsnummer:
**WO 82/02335 (22.07.82 Gazette 82/18)**

(51) Int. Cl.⁴: **A 61 F 13/20, B 65 B 63/02**

(54) **EINRICHTUNG ZUM PRESSEN VON AUS ABSORBIERENDEM MATERIAL BESTEHENDEN WERKSTÜCKEN, INSB. TAMPONS FÜR DIE FRAUENHYGIENE.**

(30) Priorität: **31.12.80 DE 3049580**

(43) Veröffentlichungstag der Anmeldung:
**29.12.82 Patentblatt 82/52**

(45) Bekanntmachung des Hinweises auf die Patenterteilung:
**02.05.85 Patentblatt 85/18**

(84) Benannte Vertragsstaaten:
**BE DE FR GB LU NL SE**

(56) Entgegenhaltungen:
**FR - A - 2 131 579**
**US - A - 3 998 031**

(73) Patentinhaber: **JOHNSON & JOHNSON GmbH,**
**Kaiserswerther Strasse 270, D-4000 Düsseldorf 30 (DE)**

(72) Erfinder: **FRIESE, Axel, Weststrasse 68,**
**D-5600 Wuppertal (DE)**

(74) Vertreter: **Groening, Hans Wilhelm, Dipl.-Ing.,**
**Siebertstrasse 4 Postfach 860 340,**
**D-8000 München 86 (DE)**

## Beschreibung

Die Erfindung betrifft eine Einrichtung zum Pressen von aus absorbierendem Material bestehenden Werkstücken, insbesondere Tampons für die Frauenhygiene, mit einer endlosen Zuführvorrichtung, die die Werkstücke vor offene Seiten einer Vorformvorrichtung und mittels mindestens eines Stössels in diese hineinbringt, einer Überführungsvorrichtung zum Überführen der vorgeformten Werkstücke von der Vorformvorrichtung nach einer Pressvorrichtung und einer Ausstossvorrichtung zum Ausstossen der gepressten Werkstücke aus der Pressvorrichtung, wobei die Vorformvorrichtung aus einer Anzahl Vorformbacken mit gegenüberliegenden Vorformgegenhaltebacken und die Pressvorrichtung aus einer Anzahl Pressbacken mit gegenüberliegenden Gegenhaltebacken besteht, wobei die Pressbacken und die Gegenhaltebacken eine im wesentlichen L-förmige Arbeitsfläche aufweisen und das Ende des kurzen Schenkels jeder Arbeitsfläche an dem langen Schenkel der gegenüberliegenden Arbeitsfläche anliegt und die kurzen Schenkel von zueinandergehörenden Arbeitsflächen derartig mit einander gegenüberliegenden Vertiefungen versehen sind, dass sie in geschlossener Stellung einen etwa zylindrischen Hohlraum bilden, wobei die Vorformbacken mit zugehörigen Vorformgegenhaltebacken und die Pressbacken mit zugehörigen Gegenhaltebacken etwa horizontal angeordnet sind und die Anzahl der Backen der Pressvorrichtung derjenigen der Vorformvorrichtung gleich ist, wobei die durch die Backen der Pressvorrichtung gebildeten Räume etwa fluchtend mit den durch die Backen der Vorformvorrichtung gebildeten Räumen an den von der Zuführvorrichtung abgelegenen offenen Seiten der Vorformvorrichtung angeordnet sind, während die Überführungsvorrichtung aus an derselben Seite wie die Zuführvorrichtung gelegenen Stösseln besteht.

Die vorgenannte Einrichtung ist in der DE-A-2 114 530 beschrieben. Ihr liegt die Aufgabe zugrunde, eine derartige Einrichtung so zu verbessern, dass sie für einen Parallelbetrieb besonders geeignet ist und der Pressvorgang so unterteilt werden kann, dass die aufzubringenden Einzelkräfte bei einer Vorformstufe und einer anschliessenden Pressstufe nach Grösse und Richtung trotzdem beherrscht werden können.

Aus der US-A-3 998 031 ist eine Vorrichtung zum Pressen und Einkapseln eines Schaumstoff-Vorformlings zur Herstellung eines eingekapselten Tampons bekannt. Dabei wird ein für die Tamponherstellung zylindrisch zugeschnittener Vorformling, der mit einem Rückholband versehen ist, in eine zylindrische Form eingesetzt und mittels eines Stössels axial gepresst sowie durch eine Öffnung in einer feststehenden Grundplatte hindurch in eine mit einer Kapsel versehene Aufnahmeöffnung in einem quer zu dieser Aufnahmeöffnung bewegbaren Schlitten hineingepresst. Die Aufnahmeöffnung ist durch einen Auswerfstössel an ihrem unteren Ende verschlossen, der senkrecht auf- und abbewegbar ist. Anschliessend wird der Schlitten in eine Krimpstation bewegt, in der die Grundplatte mit einer weiteren Durchtrittsöffnung versehen ist. Die Kapsel mit dem darin befindlichen Schaumstoff-Tampon wird durch diese weitere Durchtrittsöffnung in der Grundplatte hindurch mittens des Auswerfstössels in eine beheizte Krimpvorrichtung ausgestossen. In der Krimpvorrichtung wird der die Öffnung der den Schaumstoff-Tampon umgebenden Kapsel nach innen unter Anwendung von Hitze und Druck mittels eines beheizbaren Krimpwerkzeugs nach innen umgebördelt, so dass der Tampon in der Kapsel festgelegt ist. Die Kapsel besteht aus einem sich in Flüssigkeit auflösenden Material, so dass der Tampon nach Auflösung der Kapsel seine Funktion aufnehmen kann.

Die Erfindung sieht eine weitere Verbesserung der eingangs erwähnten Einrichtung dahingehend vor, dass aus absorbierendem Material bestehende Werkstücke, die bereits mit einem Rückholband versehen sind, ebenfalls optimal, d.h. mit einer die Massenfertigung sichernden hohen Produktionsgeschwindigkeit und hoher Betriebssicherheit sowie Qualität des Produktes, gepresst werden können, wobei insbesondere das Pressen des Werkstücks ohne Beschädigung des Rückholbandes und nach dem Pressen ein einwandfreies Andrücken des Rückholbandes an das Ende des gepressten Werkstücks, insbesondere Tampons, möglich sein soll.

Die Erfindung löst diese Aufgabe durch die im Kennzeichen des Anspruchs 1 aufgeführten Merkmale. Durch die Zwischenschaltung einer Übergabevorrichtung zwischen Vorformvorrichtung und Pressvorrichtung ist es möglich, in der Übergabestellung der Übergabevorrichtung den Vorformling aus der Vorformvorrichtung in die Pressvorrichtung und gleichzeitig einen Tampon, der sich in der Übergabevorrichtung befindet, aus dieser auszustossen, während die Übergabevorrichtung in der Aufnahmestellung einen gerade gepressten Tampon aus der Presse übernimmt, wobei diese Übernahmebewegung gleichzeitig dazu ausgenutzt wird, das Rückholband gegen das Rückholende des Tampons anzudrücken, wenn der Tampon in die Übergabevorrichtung gelangt ist. Dadurch, dass die Pressbacken und die Gegenhaltebacken der Pressvorrichtung breiter als die Gesamtlänge des Vorformlings/Tampons plus der freien Länge des daran befestigten Rückholbandes bemessen ist und die kurzen Schenkel der Pressbacken an den gegenüberliegenden Arbeitsflächen anliegen, ist eine störungsfreie Handhabung des Rückholbandes während des Fertigpressens des Vorformlings zu einem Tampon gewährleistet. Da mit der Ausstossbewegung des fertiggepressten Tampons aus der Pressvorrichtung gleichzeitig der Andrückvorgang des Rückholbandes an das Rückholende des Tampons verbunden ist, werden ein zusätzlicher Arbeitsschritt bzw. die Anordnung zusätzlicher Andrückvorrichtungen zur Durchführung dieser Arbeit erspart.

Die Ausgestaltung des Überführungskanals in der Übergabevorrichtung sowie der Überführungsvorrichtung gemäss Anspruch 2 gewährleistet eine vollständige Beaufschlagung des Vorformlings durch die Überführungsvorrichtung und damit einen störungsfreien Transport des Vorformlings in die Pressvorrichtung.

Die Ausgestaltung des Aufnahmekanals in der Übergabevorrichtung gemäss Anspruch 3 stellt sicher, dass der Stössel der Überführungsvorrichtung, der dem Querschnitt des Vorformlings entspricht, den Aufnahmekanal abschliesst und als Widerlager für den aus der Pressvorrichtung ausgestossenen Tampon dient, wenn an dessen dem Stössel der Überführungsvorrichtung abgekehrten Ende das Rückholband mittels des Stössels der Ausstossvorrichtung angedrückt wird.

Der Auswerfkanal gemäss Anspruch 4 im Gehäuse der Pressvorrichtung gewährleistet ein formschlüssiges Abführen des Tampons zu einer weiterverarbeitenden Station, z.B. Verpackungsstation.

Nachstehend ist die Erfindung anhand der schematischen Zeichnung eines Ausführungsbeispiels näher erläutert. Es zeigen:

Fig. 1 eine Ansicht der Übergabevorrichtung und Pressvorrichtung,

Fig. 2 einen Schnitt nach Linie II-II in Fig. 1,

Fig. 3 den mit dem Vorformling ausgefüllten Hohlraum zwischen den geöffneten Backen der Pressvorrichtung,

Fig. 4 den zylindrischen Hohlraum der geschlossenen Pressbacken der Pressvorrichtung mit einem darin fertig gepressten Tampon,

Fig. 5 einen Schnitt nach Linie V-V in Fig. 2 in der Übergabestellung der Übergabevorrichtung mit einem Vorformling im Überführungskanal derselben bei geöffneter Pressvorrichtung,

Fig. 6 einen Schnitt gemäss Fig. 5, in welcher der Stössel der Übergabevorrichtung den Vorformling mitsamt seines Rückholbandes vollständig in die Pressvorrichtung eingeführt hat und in die Ausgangsstellung zurückbewegt wurde,

Fig. 7 einen Schnitt gemäss Fig. 5 und 6, jedoch in der Aufnahmestellung der Übergabevorrichtung und bei geschlossener Pressvorrichtung mit fertig gepresstem Tampon und

Fig. 8 den Schnitt gemäss Fig. 7, jedoch mit dem in den Aufnahmekanal ausgestossenen Tampon, dessen Rückholband durch den Stössel der Übergabevorrichtung an das Rückholende des Tampons angepresst ist.

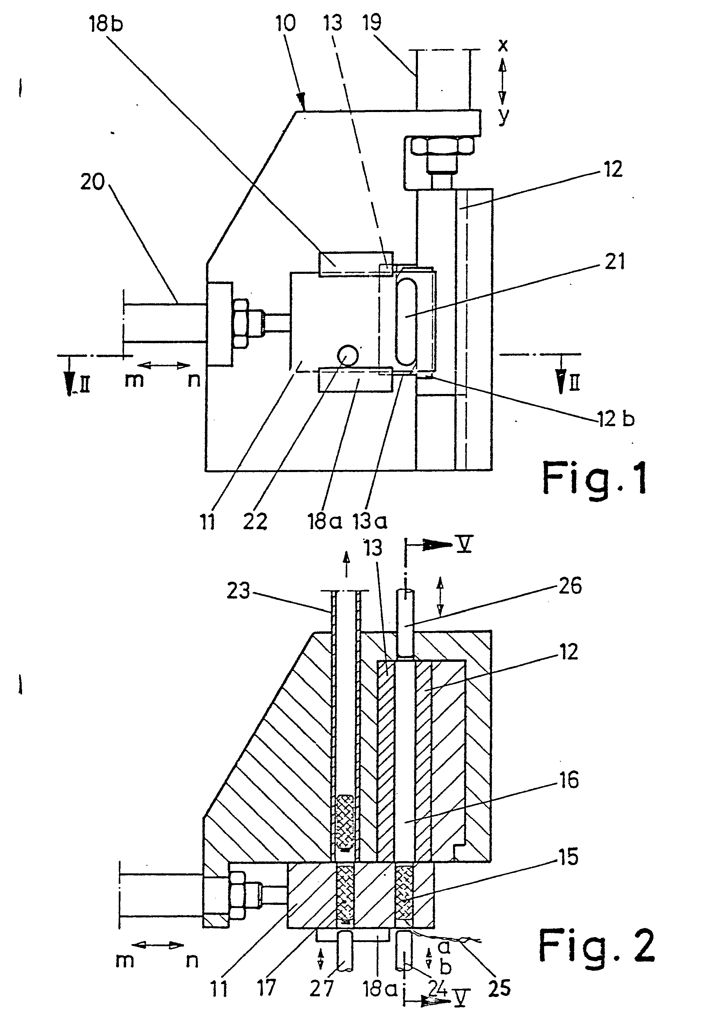

In Fig. 1 ist eine Pressvorrichtung 10 dargestellt, der eine Vorformvorrichtung vorgeschaltet ist, die in der Zeichnung aus Gründen der Vereinfachung nicht gezeigt ist. Zwischen diese Vorformvorrichtung und die Pressvorrichtung 10 ist eine Übergabevorrichtung 11 geschaltet.

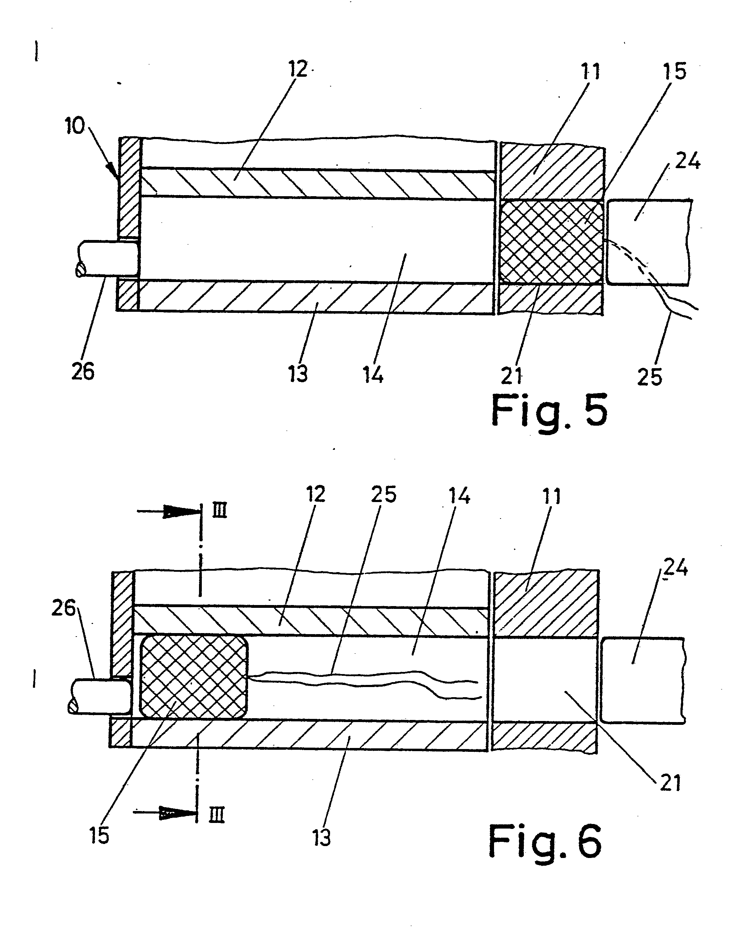

Die Pressvorrichtung 10 besteht aus einer beweglichen Pressbacke 12 und einer feststehenden Gegenhaltebacke 13. Beide Backen weisen eine im wesentlichen L-förmige Arbeitsfläche auf, deren kurzer Schenkel 12a, 13a an dem langen Schenkel 12b, 13b der gegenüberliegenden Arbeitsfläche anliegt. Die kurzen Schenkel 12a, 13a weisen zueinandergehörende Arbeitsflächen mit einander gegenüberliegenden Vertiefungen 12c, 13c auf, die in der in Fig. 5 gezeigten offenen Stellung einen im Querschnitt flachen, an den Rändern abgerundeten Hohlraum 14 für einen entsprechend geformten Vorformling 15 und gemäss Fig. 6 einen zylindrischen Hohlraum 16 für einen fertiggepressten Tampon 17 bilden.

Die Übergabevorrichtung 11 ist an ihren Längsseiten in Führungen 18a, 18b in Richtung der Pfeile m, n quer zur Bewegungsrichtung der Pressbacke 12 geführt, wobei der Antrieb für die Hin- und Herbewegung in einer an sich bekannten Weise hydraulisch und/oder mechanisch vorgesehen sein kann. Bestandteile dieser Bewegungsantriebe für die Pressbacke 12 und die Übergabevorrichtung 11 sind in Fig. 1 mit 19 und 20 angedeutet.

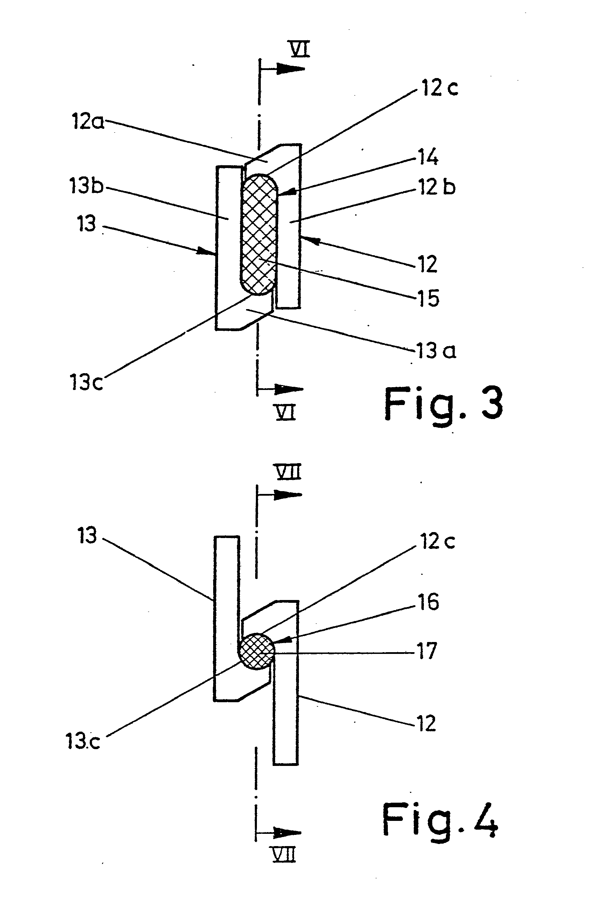

Die Übergabevorrichtung ist mit einem Überführungskanal 21 versehen, der im Querschnitt dem Hohlraum 14 der Pressbacke 12 und Gegenhaltebacke 13 entspricht, sowie ferner mit einem Aufnahmekanal 22 mit einem kreisrunden Querschnitt, dessen Durchmesser etwa dem Durchmesser des Hohlraums 16 der geschlossenen Pressbacken 12 und 13 entspricht. Ein Auswerfkanal 23 ist im Abstand parallel zu dem Überführungskanal 21 angeordnet, wie Fig. 2 zeigt, wobei der Durchmesser des Auswerfkanals der kleinen Achse des Querschnitts des Hohlraums 14 entspricht. Die an die Aussenseite des Aufnahmekanals 22 und des Überführungskanals 21 gelegte Tangentialebene verläuft parallel zur Bewegungsrichtung der Pfeile m, n der Übergabevorrichtung.

In Fig. 1 und 2 ist die Übergabevorrichtung 11 in einer Übergabestellung gezeigt, in welcher der Überführungskanal 21 mit dem Hohlraum 14 der offenen Pressbacken der Pressvorrichtung 10 fluchtet, während der Aufnahmekanal 22 mit dem Auswerfkanal 23 fluchtet, der in das Gehäuse der Pressvorrichtung 10 eingebaut ist. In Fluchtrichtung des Überführungskanals 21 und des Hohlraums 14 der offenen Pressbacken 12, 13 ist vor der nicht gezeigten Vorformvorrichtung und der Übergabevorrichtung eine als Stössel ausgebildete Überführungsvorrichtung 24 dargestellt, die in Fluchtrichtung dieser Hohlräume in Richtung der Pfeile a, b hin- und herbewegbar ist. Der Querschnitt dieser Überführungsvorrichtung 24 entspricht etwa demjenigen des Überführungskanals 21, ist aber um so viel kleiner bemessen, dass ein an dem der Überführungsvorrichtung 24 zugekehrten Rückholende des Vorformlings 15 frei herabhängendes Rückholband 25 beim Überführen des Vorformlings aus der Vorformvorrichtung in den Überführungskanal 21 der Übergabevorrichtung und in den Hohlraum der offenen Pressbacken 12, 13 nicht abgeschert oder in anderer Weise beschädigt werden kann.

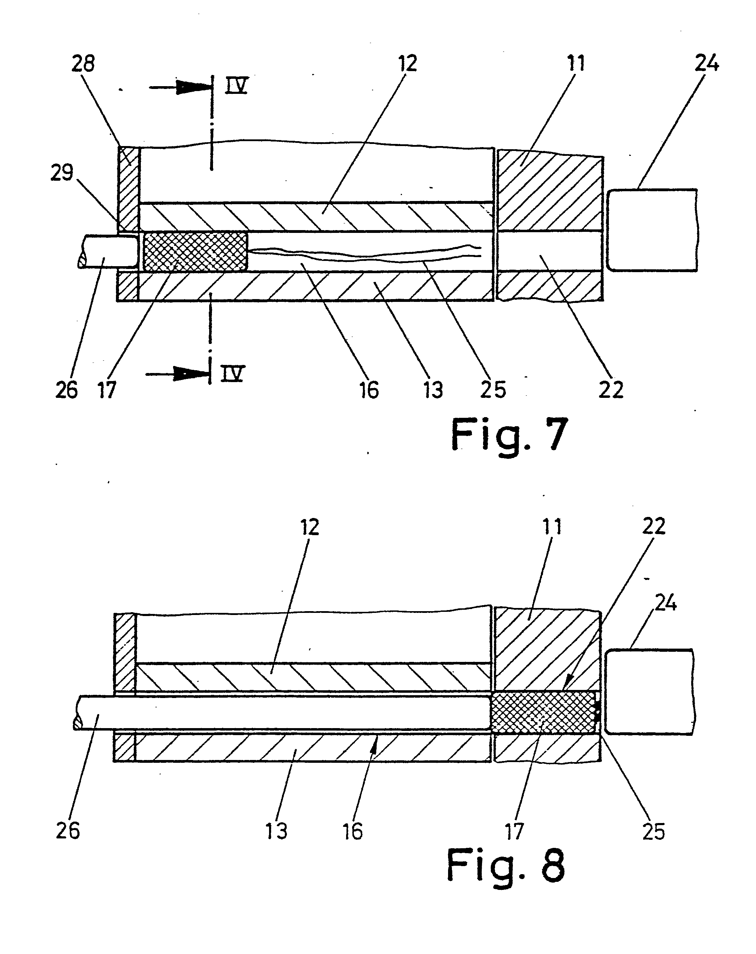

Auf der der Übergabevorrichtung 11 gegenüberliegenden Seite der Pressvorrichtung befindet sich eine als Stössel ausgebildete Ausstossvorrichtung 26, die zylindrisch ausgebildet ist und deren Querschnitt etwas kleiner als der lichte Querschnitt des Hohlraums 16 der geschlossenen Pressbacken 12, 13 der Pressvorrichtung 10 bemessen ist. Die Ausstossvorrichtung kann, wie Fig. 8 zeigt, bis zur gegenüberliegenden Öffnung des Hohlraums der Pressvorrichtung bewegt werden, um den Tampon 17 aus dem Hohlraum 16 der Pressvorrichtung heraus in den Aufnahmekanal 22 der Übergabevorrichtung zurückzustossen und dabei ein Andrücken des Rückholbandes 25 gegen das Rückholende zu bewirken, wie Fig. 8 zeigt.

In Fig. 2 ist ferner eine Auswerfvorrichtung 27 zu sehen, die koaxial zu dem Auswerfkanal 23 und dem Aufnahmekanal 22 angeordnet ist und vorzugsweise ebenfalls aus einem Stössel besteht. Diese Auswerfvorrichtung 27 dient zum Auswerfen des fertig gepressten und mit dem angedrückten Rückholband versehenen Tampons 17 durch den Auswerfkanal 23 hindurch, so dass der Tampon einer nicht gezeigten weiterverarbeitenden Station, z.B. einer Verpackungsstation, zugeführt werden kann.

Im Betrieb befindet sich die Übergabevorrichtung 11 in der in Fig. 1 und 2 gezeigten Stellung, in welcher der in der Vorformvorrichtung flachgedrückte Wattewickel als Vorformling 15 durch den Überführungskanal 21 hindurch in den Hohlraum 14 der Pressvorrichtung 10 eingeschoben wird, wobei die Pressbacken 12, 13 so breit bemessen sind, dass, wie Fig. 6 erkennen lässt, das Rückholband 25 des Vorformlings 15 in ausgestreckter Lage vollständig innerhalb des Hohlraums 14 zu liegen kommt. Die Rückseite der Presse ist durch eine Rückplatte 28 verschlossen und weist lediglich eine Durchtrittsbohrung 29 für den Stössel der Ausstossvorrichtung 26 auf. Im Anschluss an das Einführen des Vorformlings in die Pressvorrichtung 10 wird die bewegliche Pressbacke 12 in Richtung des Pfeiles y bewegt, so dass die Pressbacken die in Fig. 4 gezeigte Stellung einnehmen und der Vorformling zum Tampon 17 gepresst wird. Wie Fig. 7 zeigt, wird bei dem Pressvorgang das Rückholband 25 infolge des Entlanggleitens des kurzen Schenkels 12a an der Arbeitsfläche des langen Schenkels 13b der Gegenhaltepressbacke einwandfrei mitgenommen, ohne dass er den Pressvorgang stört oder durch den Pressvorgang beschädigt wird. Nach der Fertigstellung des Tampons wird dieser gemäss Fig. 8 mittels des Stössels der Ausstossvorrichtung 26 in den Aufnahmekanal 22 der Übergabevorrichtung 11 überführt, die in der Zwischenzeit in die Aufnahmestellung in Richtung des Pfeiles n in Fig. 1 und 2 bewegt worden ist, in welcher, wie beschrieben, der Aufnahmekanal 22 mit dem zylindrischen Hohlraum 16 der Pressbacken 12, 13 fluchtet. Die Eingangsseite des Aufnahmekanals 22 ist durch die Überführungsvorrichtung 24 verschlossen und dient als Widerlager für den Tampon, wenn dieser durch den Stössel der Ausstossvorrichtung 26 in den Aufnahmekanal 22 der Übergabevorrichtung 11 überführt und dabei das Rückholband 25 gegen das Rückholende des Tampons 17 formhaltig angedrückt werden. Zu diesem Zweck kann der Stössel der Ausstossvorrichtung 26 kurzzeitig über die normale Länge des gepressten Tampons hinaus axial bewegt werden, um eine ausreichende Andruckkraft für das Anlegen des Rückholbandes am Stirnende des Tampons zu gewährleisten. Zur Erzielung einer hinreichenden Formhaltigkeit des Rückholbandes in seinem angedrückten Zustand kann gegebenenfalls der Stössel der Überführungsvorrichtung 24 beheizt sein.

Anschliessend wird die Übergabevorrichtung wieder in die in Fig. 1 und 2 gezeigte Übergabestellung, d.h. in Richtung des Pfeiles m, zurückbewegt, so dass nunmehr der mit dem Tampon 17 gefüllte Aufnahmekanal in Fluchtrichtung des Auswerfkanals 23 liegt und der Stössel der Auswerfvorrichtung 27 den fertigen Tampon mit dem angedrückten Rückholband durch den Auswerfkanal 23 hindurch einer weiteren Arbeitsstation zuführen kann. Währenddessen wird der Stössel der Überführungsvorrichtung 24 vollständig zurückgezogen, damit er anschliessend aus der mit den Arbeitsflächen der Pressbacken fluchtenden Vorformvorrichtung einen weiteren Vorformling durch den Übergabekanal 21 in die geöffnete Pressvorrichtung 10 überführen kann.

Es ist somit ersichtlich, dass die Erfindung die Vorteile der Einrichtung gemäss der DE-A-2 114 530 auf die Verarbeitbarkeit von bereits mit einem Rückholband versehenen Tamponrohlingen auszudehnen gestattet, wobei die ausserordentlich hohe Verarbeitungsqualität und -kapazität beibehalten wird.

**Patentansprüche**

1. Einrichtung zum Pressen von aus absorbierendem Material bestehenden Werkstücken, insbesondere Tampons (17) für die Frauenhygiene, mit einer endlosen Zuführvorrichtung, die die Werkstücke vor offene Seiten einer Vorformvorrichtung und mittels mindestens eines Stössels in diese hineinbringt, einer Überführungsvorrichtung (24) zum Überführen der vorgeformten Werkstücke (15) von der Vorformvorrichtung nach einer Pressvorrichtung (10) und einer Ausstossvorrichtung (26) zum Ausstossen der gepressten Werkstücke (17) aus der Pressvorrichtung (10), wobei die Vorformvorrichtung aus einer Anzahl Vorformbacken mit gegenüberliegenden Vorformgegenhaltebacken und die Pressvorrichtung (10) aus einer Anzahl Pressbacken (12) mit gegenüberliegenden Gegenhaltebacken (13) besteht, wobei die Pressbacken (12) und die Gegenhaltebacken (13) eine im wesentlichen L-förmige Arbeitsfläche aufweisen und das Ende des kurzen Schenkels (12a, 13a) jeder Arbeitsfläche an dem langen Schenkel (12b, 13b) der gegenüberliegenden Arbeitsfläche anliegt und die kurzen Schenkel (12a, 13a) von zueinandergehörenden Arbeitsflächen derartig mit einander gegenüberliegenden Vertiefungen (12c, 13c) versehen sind, dass sie in geschlossener Stellung einen etwa zylindrischen Hohlraum (16) bilden, wobei die Vorformbacken mit zugehörigen Vorformgegenhaltebacken und die Pressbacken (12) mit zugehörigen Gegenhaltebacken (13) etwa horizontal angeordnet sind und die Anzahl Backen der Pressvorrichtung (10) derjenigen der Vorformvorrichtung gleich ist, wobei die durch die Backen (12, 13) der Pressvorrichtung (10) gebildeten Räume (14, 16) etwa fluchtend mit den durch die Backen der Vorformvorrichtung gebildeten Räumen an den von der Zuführvorrichtung abgelegenen offenen Seiten der Vorformvorrichtung angeordnet sind, während die Überführungsvorrichtung (24) aus an derselben Seite wie die Zuführvorrichtung gelegenen Stösseln besteht, dadurch gekennzeichnet, dass zwischen der Vorformvorrichtung und der Pressvorrichtung (10) eine Übergabevorrichtung (11) quer zur Bewegungsrichtung der Vorformlinge (15) bzw. Tampons (17) zwischen einer Stellung für die Übergabe der Vorformlinge (15) an die Pressvorrichtung (10) und der Tampons (17) an einen Auswerfkanal (23) sowie einer Stellung zur Aufnahme von Tampons (17) aus der Pressvorrichtung (10) hin- und herbewegbar ist, deren Pressbacken (12) und Gegenhaltebacken (13) breiter als die Gesamtlänge des Vorformlings (15) oder Tampons (17) plus der freien Länge eines daran befestigten Rückholbandes (25) bemessen sind, das gegen das Rückholende des Tampons (17) mit Hilfe der Überführungsvorrichtung (24) beim Ausstossen der Tampons (17) aus der Pressvorrichtung (10) in die Übergabevorrichtung (11) in deren Aufnahmestellung mittels der Ausstossvorrichtung (26) anpressbar ist.

2. Einrichtung nach Anspruch 1, dadurch gekennzeichnet, dass die Übergabevorrichtung (11) einen Überführungskanal (21) aufweist, der im Querschnitt demjenigen des geöffneten Hohlraums (14) der Pressvorrichtung (10) etwa entspricht und mit diesem in der Übergabestellung der Übergabevorrichtung (11) sowie mit dem Stössel der Überführungsvorrichtung (24) fluchtet, der durch den Überführungskanal (21) hindurch in den Hohlraum (14) der geöffneten Pressvorrichtung (10) koaxial hineinbewegbar ist.

3. Einrichtung nach Anspruch 1 oder 2, dadurch gekennzeichnet, dass die Übergabevorrichtung (11) einen Aufnahmekanal (22) für einen Tampon (17) aufweist, dessen Querschnitt demjenigen des geschlossenen Hohlraums (16) der Pressvorrichtung (10) entspricht und der mit dem geschlossenen Hohlraum (16) in der Aufnahmestellung der Übergabevorrichtung (11) fluchtet sowie an der Eintrittsöffnung durch den Stössel der Überführungsvorrichtung (24) verschlossen ist, welcher dem Stössel der Ausstossvorrichtung (26) auf der der Überführungsvorrichtung (24) abgekehrten Seite der Pressvorrichtung (10), in den geschlossenen Hohlraum (16) derselben koaxial hineinbewegbar gegenüberliegt.

4. Einrichtung nach einem der Ansprüche 1 bis 3, dadurch gekennzeichnet, dass der Aufnahmekanal (22) der Übergabevorrichtung (11) in der Übergabestellung derselben mit dem Auswerfkanal (23) im Gehäuse der Pressvorrichtung (10) fluchtet und die als Stössel ausgebildete Auswerfvorrichtung (27) durch den Aufnahmekanal (22) hindurch in den Auswerfkanal (23) hinein koaxial bewegbar ist.

**Claims**

1. Apparatus for pressing workpieces consisting of absorbent material, particularly tampons (17) for feminine hygiene, comprising an endless feeding device conveying the workpieces in front of open sides of a preshaping device and by means of at least one push-rod into said preshaping device; a transmitting device (24) for transmitting the pre-shaped workpieces (15) from the pre-shaping device to a pressing device (10) and an ejecting device (26) for ejecting the pressed workpieces (17) from the pressing device (10), said pre-shaping device consisting of a number of pre-shaping jaws with opposite pre-shape counterholding jaws and said pressing device (10) consisting of a number of pressing jaws (12) with opposite counterholding jaws (13), the pressing jaws (12) and the counterholding jaws (13) comprising a substantially L-shaped working surface and the end of the short leg (12a, 13a) of each working surface lying against the long leg (12b, 13b) of the opposite working surface and the short legs (12a, 13a) of associated working surface being provided with oppositely disposed recesses (12c, 13c) such that in the closed position they form an approximately cylindrical cavity (16), the pre-shaping jaws with associated pre-shape counterholding jaws and the pressing jaws (12) with associated counterholding jaws (13) being arranged about horizontally and the number of jaws of the pressing device (10) being equal to that of the pre-shaping device, the spaces (14, 16) formed by the jaws (12, 13) of the pressing device (10) being disposed approximately in alignment with the spaces formed by the jaws of the pre-shaping device at the open sides of the pre-shaping device directed away from the feeding device, while the transmitting device (24) consists of push-rods positioned on the same side as the feeding device, characterised in that, between the pre-shaping device and the pressing device (10), a delivery device (11) is adapted for reciprocating movement transversely to the direction of movement of the blanks (15) or tampons (17), respectively, between a position for the delivery of the blanks (15) to the pressing device (10) and of the tampons (17) to an ejector channel (23) and a position for receiving tampons (17) from the pressing device (10), the pressing jaws (12) and counterholding jaws (13) of said pressing device (10) being wider than the total length of the blank (15) or tampon (17) plus the free length of the withdrawal cord (25) which is attached thereto and is adapted for being pressed against the withdrawal end of the tampon (17) by means of the transmitting device (24) during the ejection of the tampon (17) from the pressing device (10) into the delivery devices (11) in the receiving position thereof by means of the ejecting device (26).

2. Apparatus according to Claim 1, characterised in that the delivery device (11) comprises a transmitting channel (21), the cross-section of which corresponds approximately to that of the open cavity (14) of the pressing device (10) and which is in alignment with said cavity in the delivery position of the delivery device (11) as well as with the phus-rod of the transmitting device (24), said push-rod being coaxially movable through the transmitting channel (21) into the cavity (14) of the opened pressing device (10).

3. Apparatus according to Claim 1 or 2, characterised in that the delivery device (11) comprises a receiving channel (22) for a tampon (17), the cross-section of which corresponds approximately to that of the closed cavity (16) of the pressing device (10) and which is in alignment with the closed cavity (16) in the receiving position of the delivery device (11) and is closed at the inlet opening by the push-rod of the transmitting device (24), said push-rod being opposite to the push-rod of the ejecting device (26) on the side of the pressing device (10) facing away from the transmitting device (24), and being coaxially movable into the closed cavity (16) of the pressing device (10).

4. Apparatus according to one of Claims 1 to 3, characterised in that the receiving channel (22) of the delivery device (11) is in the delivery position of the latter in alignment with the ejector channel (23) in the housing of the pressing device (10) and that the jecting device (27), formed as a push-rod, is coaxially movable through the receiving channel (22) into the ejector channel (23).

## Revendications

1. Dispositif pour presser des pièces à travailler constituées en un matériau absorbant, notamment des tampons (17) pour l'hygiène féminine, comportant un dispositif d'amenée sans fin, qui introduit les pièces à travailler par le côté ouvert d'un dispositif de préformage et les fait pénétrer dans ce dispositif au moyen d'au moins un poussoir, un dispositif de transport (24) servant à entraîner les pièces à travailler préformées (15) depuis le dispositif de préformage en direction d'un dispositif de pressage (10) et d'un dispositif d'éjection (26) servant à éjecter les pièces à travailler (17) pressées hors du dispositif de pressage (10), et dans lequel le dispositif de préformage est constitué par un certain nombre de mâchoires de préformage, en vis-à-vis desquelles sont disposées des mâchoires antagonistes de maintien pour le préformage et le dispositif de pressage (10) est constitué par un certain nombre de mâchoires de pressage (12) en vis-à-vis desquelles sont disposées des mâchoires antagonistes de maintien (13), et dans lequel les mâchoires de pressage (12) et les mâchoires antagonistes de maintien (13) possèdent une surface de travail essentiellement en forme de L et l'extrémité de la branche courte (12, 13a) de chaque surface de travail est appliquée contre la branche longue (12b, 13b) de la surface de travail disposée en vis-à-vis et les branches courtes (12a, 13a) de surfaces de travail réciproquement associées sont munies de renfoncements (12c, 13c) disposés réciproquement en vis-à-vis, de telle sorte que lesdites branches forment dans la position fermée, une cavité approximativement cylindrique (16), et dans lequel les mâchoires de préformage munies des mâchoires antagonistes de maintien associées et les mâchoires de pressage (12) munies des mâchoires antagonistes de maintien (13) sont disposées approximativement horizontalement et le nombre des mâchoires du dispositif de pressage est égal au nombre des mâchoires du dispositif de préformage, et dans lequel les espaces (14, 16) formés par les mâchoires (12, 13) du dispositif de pressage (10) sont disposés de manière à être approximativement alignés avec les espaces, formés par les mâchoires du dispositif de préformage, sur les côtés ouverts de ce dispositif, qui sont situés à l'opposé du dispositif d'amenée, tandis que le dispositif de transport (24) est constitué par des poussoirs situés sur le même côté que le dispositif d'amenée, caractérisé par le fait qu'un dispositif de transfert (11) peut être déplacé selon un mouvement de va-et-vient, entre le dispositif de préformage, et le dispositif de pressage (10) transversalement par rapport à la direction de déplacement des préformes (15) ou des tampons (17), entre une position prévue pour le transfert des préformes (15) au dispositif de pressage (10) et pour le transfert des tampons (17) à un conduit d'éjection (23) et une position de réception permettant le prélèvement de tampons (17) hors du dispositif de pressage (10), dont les mâchoires de pressage (12) et les mâchoires antagonistes de maintien (13) possèdent une largeur supérieure à la longueur totale de la préforme (15) ou du tampon (17) plus la longueur libre d'une bande de récupération (25) qui est fixée à la préforme ou au tampon et qui peut être repoussée à l'aide du dispositif d'éjection (26), contre l'extrémité de récupération du tampon (17) à l'aide du dispositif de transport (24) lors de l'expulsion du tampon (17) depuis le dispositif de pressage (10) dans le dispositif de transfert (11), dans la position de réception.

2. Dispositif selon la revendication 1, caractérisé en ce que le dispositif de transfert (11) comporte un conduit de transport (21) dont la section transversale correspond approximativement à celle de la cavité ouverte (14) du dispositif de pressage (10) et est alignée avec celle-ci lorsque le dispositif de transfert (11) est dans sa position de transfert, ainsi qu'avec le poussoir du dispositif de transport (24), qui peut être entraîné coaxialement, à travers le conduit de transport (21), dans la cavité (14) du dispositif de pressage (10) ouvert.

3. Dispositif selon la revendication 1 ou 2, caractérisé en ce que le dispositif de transfert (11) comporte un conduit (22) de réception pour un tampon (17), dont la section transversale correspond approximativement à celle de la cavité fermée (16) du dispositif de pressage (10) et qui est alignée avec la cavité fermée (16), lorsque le dispositif de transfert (11) est dans la position de réception, et qui est fermé, au niveau de l'ouverture d'entrée, par le poussoir du dispositif de transport (24), conduit de réception (22) en regard duquel est disposé le poussoir du dispositif d'éjection sur le côté du dispositif de pressage (10), situé à l'opposé du dispositif de transport (24), de manière à être déplaçable coaxialement dans la cavité fermée (16) de ce dispositif de pressage.

4. Dispositif selon l'une des revendications 1 à 3, caractérisé par le fait que lorsque le dispositif de transfert (11) est dans sa position de transfert, le conduit de réception (22) de ce dispositif est aligné avec le conduit d'éjection (23) situé dans le carter du dispositif de pressage (10) et que le dispositif d'éjection (27) réalisé sous la forme d'un poussoir peut être déplacé à travers le conduit de réception (22), de manière à être introduit coaxialement dans le conduit d'éjection (23).

0 067 834

Fig.1

Fig. 2

VI

12 c

12a

13b
14
12 b

13
12

15

13a

13c

# Fig. 3

VI

VII

13

12 c

16

17

12

13.c

# Fig. 4

VII

**Fig. 5**

**Fig. 6**

Fig. 7

Fig. 8